(19) Europäisches Patentamt
European Patent Office
Office européen des brevets

(11) Veröffentlichungsnummer: **0 003 315 B1**

(12) **EUROPÄISCHE PATENTSCHRIFT**

(45) Veröffentlichungstag der Patentschrift: **30.09.81**

(51) Int. Cl.³: **C 08 F 4/00** //C07C121/40, C07C121/28

(21) Anmeldenummer: **79100129.0**

(22) Anmeldetag: **17.01.79**

# (54) Verwendung reaktiver Oligomerer zur Härtung ungesättigter Polyesterharze.

(30) Priorität: **27.01.78 DE 2803494
13.02.78 DE 2805904**

(43) Veröffentlichungstag der Anmeldung:
**08.08.79 Patentblatt 79/16**

(45) Bekanntmachung des Hinweises auf die Patenterteilung:
**30.09.81 Patentblatt 81/39**

(84) Benannte Vertragsstaaten:
**DE FR GB IT**

(56) Entgegenhaltungen:
**BE - A - 646 953
DE - A - 2 030 589
DE - A - 2 033 910
DE - A - 2 444 252**

(73) Patentinhaber: **BAYER AG
Zentralbereich Patente, Marken und Lizenzen
D-5090 Leverkusen 1, Bayerwerk (DE)**

(72) Erfinder: **Braun, Dietrich, Dr.
Jakob-Jung-Strasse 56
D-6100 Darmstadt-Arheilgen (DE)**
Erfinder: **Titzschkau, Klaus, Dr.-Ing.
Teutoburger Strasse 13
D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 315 B1

Verwendung reaktiver Oligomerer zur Härtung ungesättigter Polyesterharze

Die Erfindung betrifft die Verwendung reaktiver Oligomerer aus $\alpha$-substituierten Acrylsäurederivaten und Tetraarylbernsteinsäurenitrilen als Initiatoren für die Härtung ungesättigter Polyesterharze.

Gegenstand der Erfindung ist die Verwendung reaktiver Oligomerer der Formel

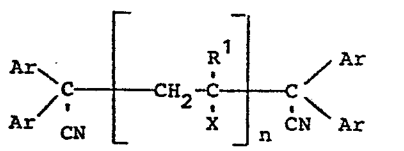 (I)

worin
Ar Phenyl, $C_1$—$C_4$-Alkylphenyl oder Chlorphenyl,
$R^1$ Alkyl mit 1 bis 4 C-Atomen,
X $COOR^2$ oder CN ($R^2$ = Alkyl mit 1 bis 12 C-Atomen oder ein Wasserstoffatom) und
n eine Zahl von 1 bis 20 bedeuten.

Bevorzugt zu verwendende reaktive Oligomere sind solche der Formel I, worin $R^1$ Methyl, $R^2$ H, Methyl oder Ethyl; X = $COOR^2$ und n eine Zahl von 2 bis 6 bedeuten.

Besonders bevorzugt verwendet werden reaktive Oligomere aus Tetraphenylbernsteinsäuredinitril und Methylmethacrylat mit 2 bis 6 Methylmethacrylatresten.

Die BE—PS 646 953 betrifft sogenannte "stabile" Präpolymere, die durch Reaktion eines ethylenisch ungesättigten Monomeren, z.B. eines Methacylsäureesters (Seite 6, Absatz 2), mit einer verhältnismäßig großen Menge eines Radikalbildners vom Typ des Tetraphenylbernsteinsäuredinitrils erhalten werden. Durch Energiezufuhr können diese "stabilen" Präpolymeren in vollständig ausgehärtete Polymere überführt werden. Im Beispiel 1 der Tabelle 2 wird ein Copolymerisat aus Styrol und Divinylbenzol in Gegenwart von Tetraphenylbernsteinsäuredinitril hergestellt und dessen Viskositätsänderung während mehrwöchiger Lagerzeit gemessen. Es kann dieser Literaturstelle nicht entnommen werden, daß sich die "stabilen" Präpolymeren als Initiatoren für ungesättigte Polyesterharze eignen.

Die DE—OS 20 30 589 betrifft telechele Oligomere der Formel

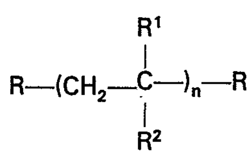

worin R das Radikalbruchstück einer symmetrischen Azoverbindung bedeutet. Substituenten R, die zwei Arylreste enthalten, werden in der Literaturstelle nicht erwähnt. Die telechelen Oligomeren der DE—OS 20 30 589 können als Zwischenprodukt in der Kunststoffindustrie, als Weichmacher, Hydraulikflüssigkeiten, Maschinenöle, Zusätze zu Anstrichmitteln und als Formulierungshilfsmittel verwendet werden; vgl. Seite 17. Auch die DE—OS 20 30 589 gibt also keinen Hinweis, daß die erfindungsgemäß zu verwendenden telechelen Oligomeren hervorragende Initiatoren für ungesättigte Polyesterharze sind.

Betrachtet man die oben besprochenen Literaturstellen, muß man feststellen, daß gemäß der vorliegenden Anmeldung aus einer großen Anzahl von Radikalbildnern und ungesättigten Monomeren jeweils wenige Verbindungen ausgewählt werden, aus denen dann das telechele Oligomere aufgebaut wird. Andererseits werden unter einer großen Anzahl polymerisationsfähiger Systeme solche eines ganz speziellen Typs, nämlich Mischungen ungesättigter Polyesterharze und damit copolymerisierbarer Monomerer, ausgewählt. Ungesättigte Polyesterharze und rein monomere Systeme, wie z.B. (Meth-)Acrylsäure, unterscheiden sich aber in Reaktivität und Lagerstabilität so weitgehend voneinander, daß sich Erfahrungen, die man mit den Monomeren macht, auf ungesättigte Polyesterharze nicht ohne weiteres übertragen lassen.

Die Oligomeren der Formel (I) können aus den entsprechenden $\alpha$-substituierten Acrylsäurederivaten und den Tetraarylbernsteinsäuredinitrilen erhalten werden. Bevorzugte $\alpha$-substituierte Acrylsäurederivate entsprechen der Formel (II)

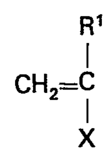 (II)

in der
$R^1$ und X die bereits angegebene Bedeutung haben.

Besonders bevorzugt ist Methylmethacrylat.

Bevorzugte Tetraarylbernsteinsäuredinitrile entsprechen der Formel (III)

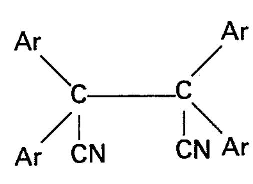 (III)

in der
Ar die bereits angegebene Bedeutung hat.

Zur Herstellung der Oligomeren kann man das Tetraarylbernsteinsäuredinitril in dem $\alpha$-substituierten Acrylsäurederivat lösen oder suspendieren und diese Mischung dann auf

60—100°C erwärmen. Mann kann auch in Anwesenheit eines inerten Verdünnungsmittels arbeiten, z.B. in gegebenenfalls halogenierten, aromatischen Kohlenwasser-stoffen wie Benzol, Chlorbenzol, Xylol, Toluol.

Die einzusetzende Menge Tetraarylbernsteinsäuredinitril, bezogen auf das $\alpha$-substituierte Acrylsäurederivat, richtet sich nach dem gewünschten Oligomerisationsgrad des reaktiven Oligomeren I. Je mehr Tetraarylbernsteinsäuredinitril, bezogen auf das Acrylsäurederivat, eingesetzt wird, desto geringer wird der Oligomerisationsgrad.

Die einzusetzende Menge Tetraarylbernsteinsäuredinitril kann etwa 40 bis etwa 0,05 Gew.-%, bezogen auf $\alpha$-substituiertes Acrylsäurederivat, betragen. Wenn man ohne Verdünnungsmittel arbeitet, d.h. den Überschuß des $\alpha$-substituierten Acrylsäurederivats als Reaktionsmedium verwendet, entspricht dies Oligomerisationsgraden von etwa 2 bis etwa 20. Verwendet man kleinere Mengen Tetraarylbernsteinsäuredinitril — etwa bis 1 Gew.-%, bezogen auf $\alpha$-substituiertes Acrylsäurederivat — dann kann nach Ende der Oligomerisierung erneut Tetraarylbernsteinsäuredinitril zum Ansatz gegeben werden, worauf sich weiter Oligomeres bildet. Dies kann so lange wiederholt werden, als sich noch neues Oligomeres im $\alpha$-substituierten Acrylsäurederivat löst. Meist liegt eine praktische Grenze bei etwa 20 bis 30% Umsatz des $\alpha$-substituierten Acrylsäurederivats.

Die Oligomeren können durch Fällung mit geeigneten Fällmitteln (Petrolether) isoliert werden oder auch durch destillative Entfernung der Restmonomeren.

Die erhaltenen reaktiven Oligomeren sind im allgemeinen bei Zimmertemperatur feste Stoffe. Sie sind unempfindlich gegen Luft und Licht, leicht löslich in einer Reihe von Vinylmonomeren und Lösungsmitteln (Alkohole, Aromaten, Chloroform, Aceton, Tetrahydrofuran, Dimethylformamid), aber unlöslich in Wasser und Petrolether. Ihre hervorstechendste Eigenschaft ist, daß sie selbst reaktiv sind, d.h. Radikalbildnereigenschaften besitzen.

Es ist erstaunlich, daß sie überhaupt exitenzfähig und sogar stabil sind, denn Tetraphenylbernsteinsäuredinitril ist als Initiator für die Polymerisation methylenischer Monomerer bekannt. Es wäre demnach zu erwarten daß sich in Anwesenheit dieses Initiators Polymere bilden. Dies ist mit den erfindungsgemäßen $\alpha$-substituierten Acrylsäurederivaten allerdings erstaunlichweise nicht der Fall. Es bilden sich Oligomere auch dann, wenn das Monomer in großem Überschuß vorhanden ist.

Substrat für die Polymerisationsinitiierung mit den erfindungsgemäß zu verwendenden reaktiven Oligomeren sind ungesättigte Polyesterharze, d.h. die Lösungen $\alpha,\beta$-ethylenisch ungesättigter Polyester in damit copolymerisierbaren monomeren Vinyl- oder Vinylidenverbindungen. Im allgemeinen liegt das Polyester/Monomer-Verhältnis zwischen 30/70 und 70/30 Gewichtsteilen.

Bevorzugte $\alpha,\beta$-ethylenisch ungesättigte Polyester sind die üblichen Polykondensationsprodukte mindestens einer $\alpha,\beta$-ethylenisch ungesättigten Dicarbonsäure mit in der Regel 4 oder 5 C-Atomen oder deren esterbildenden Derivate, z.B. ihren Anhydriden, gegebenenfalls in Abmischung mit bis zu 200 Mol-%, bezogen auf die ungesättigten Säurekomponenten, mindestens einer aliphatischen gesättigten Dicarbonsäure mit 4 bis 10 C-Atomen oder einer cycloaliphatischen oder aromatischen Dicarbonsäure mit 8—10 C-Atomen oder deren esterbildenden Derivate mit mindestens einer Polyhydroxyverbindung, insbesondere Dihydroxyverbindung, mit 2—8 C-Atomen — also Polyester, wie sie bei J. Björksten et. al., "Polyesters an their Applications", Reinhold Publishing Corp., New York 1956, beschrieben sind.

Beispiele für bevorzugt zu verwendende ungesättigte Dicarbonsäuren oder ihre Derivate sind Maleinsäure oder Maleinsäureanhydrid und Fumarsäure. Verwendet werden können z.B. jedoch auch Mesaconsäure, Citraconsäure, Itaconsäure oder Chlormaleinsäure. Beispiele für die zu verwendenden aliphatischen gesättigten, cyclo-aliphatischen und aromatischen Dicarbonsäuren oder ihre Derivate sind Phthalsäure oder Phthalsäure-anhydrid, Isophthalsäure, Terephthalsäure, Hexa- oder Tetrahydrophthalsäure bzw. deren Anhydride, Endomethylentetrahydrophthalsäure oder deren Anhydrid, Bernsteinsäure bzw. Bernsteinsäureanhydrid und Bersteinsäureester und -chloride, Adipinsäure, Sebacinsäure. Um schwerentflammbare Harze herzustellen, können z.B. Hexachlorendomethylentetrahydrophthalsäure, Tetrachlorphthalsäure oder Tetrabromphthalsäure verwendet werden. Als zweiwertige Alkohole können Äthylenglykol, Propandiol-1,2, Propandiol-1,3, Diäthylenglykol, Dipropylenglykol, Butandiol-1,3, Butandiol-1,4, Neopentylglykol, Hexandiol-1,6, 2,2-Bis-(4-hydroxycyclohexyl)-propan, bis-oxalkyliertes Bisphenol A, Perhydrobisphenol und andere eingesetzt werden. Bevorzugt verwendet werden Äthylenglykol, Propandiol-1,2, Diäthylenglykol und Dipropylenglykol.

Weitere Modifikationen sind möglich durch Einbau ein-, drei- und vierwertiger Alkohole mit 1—6 C-Atomen, wie Methanol, Äthanol, Butanol, Allylalkohol, Benzylalkohol, Cyclohexanol und Tetrahydrofurfurylalkohol, Trimethylolpropan, Glycerin und Pentaerythrit sowie von Mono-, Di- und Triallyläthern und Benzyläthern drei- und mehrwertiger Alkohole mit 3—6 C-Atomen gemäß DE-AS 1 024 654, sowie durch Einbau einbasischer Säuren wie Benzoesäure, oder langkettiger ungesättigter Fettsäuren wie Ölsäure, Leinölfettsäure und Ricinenfettsäure.

Die Säurezahlen der Polyester liegen gewöhnlich zwischen 1 und 100, vorzugsweise zwischen 20 und 70, die OH-Zahlen zwischen

10 und 150, vorzugsweise zwischen 20 und 100, und die als Zahlenmittel bestimmten Molekulargewichte $\bar{M}_n$ zwischen ca. 500 und 5000, vorzugsweise zwischen ca. 1000 und 3000 liegen (dampfdruckosmometrisch gemessen in Dioxan und Aceton; bei differierten Werten wird der niedrigere als der korrekte angesehen).

Als mit den ungesättigten Polyestern copolymerisierbare Vinyl- und Vinylidenverbindungen eignen sich in der Polyestertechnologie gebräuchliche ungesättigte Verbindungen, die bevorzugt $\alpha$-substituierte Vinyl-oder Vinylidengruppen oder $\beta$-substituierte Allylgruppen tragen, bevorzugt Styrol; aber auch beispielsweise kernchlorierte und -alkylierte bzw. -alkenylierte Styrole, wobei die Alkylgruppen 1—4 Kohlenstoffatome enthalten können, z.B. Vinyltoluol, Divinylbenzol, $\alpha$-Methylstyrol, tert.-Butylstyrol, Chlorstyrole; Vinylester von Carbonsäuren mit 2—6 Kohlenstoffatomen, bevorzugt Vinylacetat; Vinylpyridin, Vinylnaphthalin, Vinylcyclohexan, Acrylsäure und Methacrylsäure und/oder ihre Ester (vorzugsweise Vinyl-, Allyl- und Methallylester) mit 1—4 Kohlenstoffatomen in der Alkoholkomponente, ihre Amide und Nitrile, Maleinsäureanhydrid, -halb- und -dieseter mit 1—4 Kohlenstoffatomen in der Alkoholkomponente, -halb- und -diamine oder cyclische Imide wie N-Methylmaleinimid oder N-Cyclohexylmaleinimid; Allylverbindungen wie Allylbenzol und Allylester wie Allylacetat, Phthalsäureidallylester, Isophthalsäurediallylester, Fumarsäurediallylester, Allylcarbonat, Diallylcarbonat, Triallyphosphat und Triallylcyanurat.

Aber auch Polyester, die frei von copolymerisierbaren Vinyl- oder Vinylidenverbindungen sind, lassen sich erfindungsgemäß in Gegenwart der reaktiven Oligomeren härten. In solchen Fällen handelt es sich um sogenannte "lufttrocknende" Polyester, die $\beta,\alpha$-ethylenisch ungesattigte Ethergruppen im allgemeinen in Mengen von 0,2 bis 0,8 Mol pro 100 g Polyester, enthalten. Derartige Polyester sind z.B. in der DE-PS 22 21 335 beschrieben.

Die erfindungsgemäßen reaktiven Oligomeren können in Mengen von 0,05 bis 3, vorzugsweise 0,1 bis 2, Gew.-%, bezogen auf ungesättigtes Polyesterharz, eingesetzt werden.

Der Beginn der Polymerisationsreaktion wird durch Erhitzen einer Mischung aus ungesättigtem Polyesterharz und reaktivem Oligomeren über eine konkrete, im Einzelfall leicht zu bestimmende Anspringtemperatur erreicht.

Die Härtung der ungesättigten Polyesterharze kann in der Regel zwischen 60 und 200°C erfolgen.

Gegenüber den Initiatoren des Standes der Technik besitzen die erfindungsgemäßen reaktiven Oligomeren bzw. die bei der Polymerisationsinitiierung entstehenden Folgeprodukte den Vorzug, den Polymerisationsschwund zu vermindern und nach der Härtung nicht aus dem Polymerisat auszudiffundieren.

Die in Gegenwart der erfindungsgemäßen reaktiven Oligomeren gehärteten Polymerisate besitzen sehr gute Shore-Härten und zeigen auch im Beige- und Schlagbiegeversuch hervorragende Werte.

Die nachfolgenden Beispiel erläutern die Erfindung.

Beispiel 1

Herstellung des Oligomeres

In einem 100 ml Dreihalskolben mit Rührer und Rückflußkühler werden unter Stickstoff 10 g Tetraphenylbernsteinsäuredinitril und 38,4 g Methylmethacrylat auf 90°C erwärmt. Man erhält eine Suspension des Dinitrils mit Methylmethacrylat, da sich das Dinitril nicht vollständig löst. Nach 6 Stunden ist kein Bodenkörper mehr vorhanden; das Oligomer aus Methylmethacrylat und Tetraphenylbernsteinsäuredinitril hat sich im überschüssigen Monomeren aufgelöst. Nach einer weiteren Stunde läßt sich aus einer der Reaktionslösung entnommenen Probe mit Methanol nichts ausfällen. Erst nach einer weiteren halben Stunde gelingt es, geringe Mengen Polymethylmethacrylat auszufällen, während freies Tetraphenylbernsteinsäuredinitril praktisch nicht mehr vorhanden ist. Die Reaktion wird abgebrochen, durch Kühlen auf etwa 20°C und das nicht umgesetzte Methylmethacrylat im Vakuum abdestilliert. Aus dem Rückstand wird Oligomer mit Methanol extrahiert und durch Eindampfen in reiner Form gewonnen. Ausbeute 17,2 g; Oligomerisationsgrad 2,8.

Beispiel 2

In einem 250 ml Dreihalskolben mit Rührer und Rückflußkühler werden unter Stickstoff 106 mg Tetraphenylbernsteinsäuredinitril und 94 g Methylmethacrylat auf 90°C erhitzt. Das Tetraphenylbernsteinsäuredinitril löst sich vollstandig auf. Im Abstand von einigen Minuten werden weitere Chargen von je 106 mg Tetraphenylbernsteinsäuredinitril zugegeben, die folgende Charge immer nach vollständiger Auflösung der letzten Charge. Dieser Vorgang wird 21mal wiederholt. Die Reaktionsmischung wird dann wie in Beispiel 1 aufgearbeitet. Man erhält allerdings 2 Oligomerfraktionen, von denen eine in Methanol löslich und die andere in Methanol unlöslich ist. Die methanollösliche Fraktion 9,3 g besitzt einen Oligomerisationsgrad von ca. 15, die methanolunlösliche Fraktion 3,7 g einen solchen von ca. 30 Insgesamt werden 13 g Oligomer mit einem mittleren Oligomerisierungsgrad von 19,75 erhalten.

Beispiel 3

Härtung ungesättigtes Polyesterharze

Ein hochreaktives Gemisch, enthaltend einen Maleinsäureanhydrid / Phthalsäureanhydrid / Propylenglykol - Polyester (Molverhältnis MSA/PSA 70:30) und Styrol, mit einem Styrolgehalt von 34 Gew.-%, einer bei 20°C bestimmten Viskosität von 1250 mPa.s. und einem Doppelbindungsgehalt von 0,22 Aquivalenten

Fumarsäure pro 100 g ungesättigten Polyester, wird nachfolgend als Polyesterharz UPE bezeichnet.

Das Polyesterharz UPE wurde mit den unten aufgeführten Mengen reaktiver Oligomerer gemischt, und diese Mischung in Reagenzgläser mit einem Durchmesser von 2 cm, 8 cm hoch, eingefüllt. Die Härtung erfolgte bei einer Badtemperatur von 110°C, wobei die Maximaltemperatur im Polyesterharz entsprechend DIN 16 945 mit einem Eisen/Konstantan-Thermoelement verfolgt wurde. Man erhielt einwandfrei ausgehärtete Produkte.

| | Menge des Oligomeren in Gew.-% bez. auf UPE | erreichte Maximaltemperatur [°C] |
|---|---|---|
| Oligomeres aus Beispiel 1 | 0,5 | 209 |
| | 1,0 | 214 |
| | 1,5 | 216 |
| Oligomeres aus Beispiel 2 | 0,5 | 208 |
| | 1,0 | 219 |
| | 1,5 | 224 |

**Patentanspruch**

Verwendung von Oligomeren der Formel

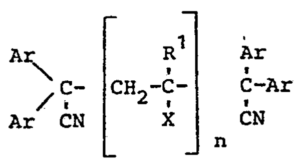

worin
Ar Phenyl, $C_1$—$C_4$-Alkylphenyl oder Chlorphenyl,
$R^1$ Alkyl mit 1 bis 4 C-Atomen,
X für $COOR^2$ oder CN,
$R^2$ für H oder Alkyl mit 1 bis 12 C-Atomen und
n für eine Zahl vom 1 bis 20 stehen,
zur Härtung ungesättigter Polyesterharze.

**Claim**

Use of oligomers of the formula

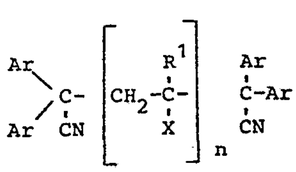

wherein
Ar represents phenyl, $C_1$—$C_4$ alkylphenyl or chlorophenyl,
$R^1$ represents alkyl with 1 to 4 C atoms,
X represents $COOR^2$ or CN,
$R^2$ represents H or alkyl with 1 to 12 C atoms and
n represents a number from 1 to 20,
for curing unsaturated polyester resins.

**Revendication**

Utilisation d'oligomères de formule

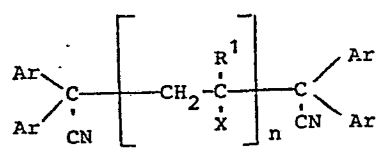

dans laquelle Ar représente un groupe phényle, (alkyl en $C_2$—$C_4$)— phènyle ou chlorophényle; $R^1$ un groupe alkyle en $C_1$—$C_4$; X représente $COOR^2$ ou CN; $R^2$ représente H où un alkyle en $C_1$—$C_{12}$; et n représente un entier de 1 à 20; pour le durcissement de résines polyesters insaturées.